(19)

**Europäisches Patentamt**
**European Patent Office**
**Office européen des brevets**

(11)  **EP 4 461 221 A1**

(12)  # EUROPEAN PATENT APPLICATION

(43) Date of publication:
**13.11.2024  Bulletin 2024/46**

(51) International Patent Classification (IPC):
**A61B 5/305** [(2021.01)]

(21) Application number: **23382433.3**

(52) Cooperative Patent Classification (CPC):
**A61B 5/305**

(22) Date of filing: **10.05.2023**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Universidad de Granada**
**18071 Granada (ES)**

(72) Inventors:
• **DE LA TORRE VEGA, Ángel**
**18071 Granada (ES)**

• **ÁLVAREZ RUIZ, Isaac Manuel**
**18071 Granada (ES)**
• **SEGURA LUNA, José Carlos**
**18071 Granada (ES)**
• **VALDERRAMA VALENZUELA, Joaquín Tomás**
**18071 Granada (ES)**
• **PALMA LÓPEZ, Alberto José**
**18071 Granada (ES)**
• **CARVAJAL RODRÍGUEZ, Miguel Ángel**
**18071 Granada (ES)**
• **POUSIBET GARRIDO, Antonio**
**18071 Granada (ES)**

(74) Representative: **TRBL Intellectual Property**
**Glorieta de Quevedo, 8**
**28015 Madrid (ES)**

(54)  **DEVICES AND METHODS FOR COMMON MODE NOISE REDUCTION IN BIOPOTENTIAL ACQUISITIONS**

(57)  The present invention discloses a device and method for reducing common mode noise in biopotential acquisition processes. The device comprises a first and a second (1, 1') electrodes connected to a first differential amplifier, a second differential amplifier (3') connected to a third electrode (1") acting as a ground and to averaging means (5) for averaging the signals coming from the first and second electrodes (1, 1'); means for combining (8) the first and second output signals (4, 4') coming from the first and second differential amplifiers (3, 3'), the second output signal being weighted by a gain factor (7), gain factor control means (11) and a registration unit (10). A method is also disclosed such that the described device is used to minimise the common mode noise component of a biopotential acquisition signal by adjusting the value and sign of the gain factor (7).

FIG. 1

EP 4 461 221 A1

**Description**

**FIELD OF THE INVENTION**

The present invention belongs to the field of biopotential acquisition systems, that is, acquisition of electrical potentials generated by biological systems. More specifically, the invention describes a method for reducing common mode electrical noise (mainly associated with external electrical interference, such as interference from a power supply network, etc.) that generally affects the electrical signals of biopotentials, and thus improve the quality of the acquired biopotentials. Specific implementations of said method are also disclosed, as well as the device that implements them.

**BACKGROUND OF THE INVENTION**

Some physiological processes, such as neuronal and/or muscular activity, produce naturally electrical signals called biopotentials. These biopotentials are of special relevance in medicine, biology and other fields since they can be measured and used to study and/or monitor biological activity. Among these biopotentials, those most commonly known are generated by the heart and measured via electrocardiography (ECG) (also foetal electrocardiography (FECG)), by the brain activity and measured via electroencephalography (EEG) or by the muscles and measured via electromyography (EMG).

A special case of biopotentials is that of the so-called evoked potentials, which are characterised by a specific pattern and are recorded from a particular part of the nervous system (e.g. auditory nerve and/or auditory pathway in the case of auditory evoked potentials; visual nerve and/or visual pathway in the case of visual evoked potentials) after delivering one or more stimuli, in such a way that different types of potentials result from stimuli of different types. Evoked potential amplitudes are typically low, ranging from less than one microvolt to a few microvolts, whereas other biopotential amplitudes can reach several millivolts. For instance, EEG usually corresponds to tens of microvolts, EMG to a few millivolts, and ECG to even 20 or more millivolts.

Some of these evoked potentials are more challenging to register than others, due to their typically low amplitudes. Different sources of noise affect considerably their measurement, being these sources external (such as interference from a power supply network with the measuring apparatus or with the subject under exploration) or internal (other biopotentials that might be captured as background). A particularly relevant case is that of the auditory evoked response (AER), which is the electrical activity of the nervous system in response to an auditory stimulus. This electrical activity is characterised by a number of voltage peaks of very low amplitude which are generated in different parts of the auditory pathway, and that can be classified according to their generator site and the time between stimulus onset and occurrence of the peaks (peak latency), which ranges from 1 ms to 0.5 s.

The acquisition of good-quality biopotentials, especially in the case of (auditory) evoked potentials, requires adequate amplification of the electrical signal (using low-noise instrumentation amplifiers) at electrodes located near the biopotential generating centres. The quality of these acquired biopotentials depends on the characteristics of the electrical signals, such as the amplitude of the potentials in the acquisition electrodes, which are dependent, in turn, on the following factors:

- the nature of the biopotentials and the proximity of the electrodes to the generating centres;
- the electrical impedance on the signal source, which depends on the generating centres, the type of electrodes, their location in relation to the generating centres and the contact impedance of the electrodes; and
- the noise conditions that affect the acquisition procedure, including exogenous noise sources, due to external interference, and/or endogenous noise sources, associated with electrical activity or biopotentials other than those of interest in a specific acquisition.

To obtain good quality biopotential recordings, low noise differential amplifiers are used, with equivalent internal noise level at the input of the amplifier below 1 $\mu$V rms, or even lower. Another important characteristic of said amplifiers is their common mode rejection ratio (CMRR), which must be sufficiently high in order to obtain good quality measurements of the biopotentials of interest. The reason for this is that a differential amplifier with a high CMRR value substantially reduces common mode noise, mainly caused by external interference, which acts as the main source of noise affecting biopotential acquisition systems.

Nevertheless, the acquisition of a high-quality biopotential recording requires the contact impedance of the electrodes to be low (to reduce noise and avoid gain reduction) and, most importantly, it must be well balanced between the electrodes. Also, an environment free of electrical noise (e.g., in a shielded room) is required to avoid electromagnetic interference. The first condition is needed because if the electrodes used for the recording present unbalanced impedances, the result is an important increment of the common mode gain. The second one allows to eliminate as many external electrical interferences as possible.

However, in practice, having a quality shielded room makes the biopotential acquisition process substantially more expensive. Furthermore, the patient or subject must be moved to a designated room in order to perform the recording, limiting the applicability of the acquisition. On the other hand, reducing electrode impedance can be achieved by replacing surface electrodes on the skin with subcutaneous or intramuscular needle electrodes, but at the cost of making the scan a much more invasive process. Moreover, this does not guarantee a good balance between the electrodes' contact impedances.

On the other hand, the use of surface electrodes instead of needle electrodes, although much more economic and patient-friendly, results in contact impedances of the order of a few kiloohms which are, typically, not perfectly balanced. This, together with the use of a low-quality shielded room or with the unavailability of a shielded room at all, gives rise to very strong common mode components in the acquired signals, reducing their final quality.

Some methods, such as the so-called Driven Right Leg (DRL) technique, aim to ameliorate these effects by implementing specifically built electric circuits comprising differential amplifiers to reduce common-mode interference. The DRL improves the CMRR siphoning away part of the common mode voltage that the human body picks up from nearby power lines or other electrical devices. Nevertheless, its results are limited when dealing with low-amplitude signals, such as those from evoked potentials.

Therefore, it is of great interest, given its importance in medical and scientific applications, to provide a solution that can reach enough common mode reduction levels such that good quality biopotential recordings can be obtained. Specifically, it is particularly relevant to do so such that the use of non-expensive (e.g., not using electromagnetically shielded rooms) and non-invasive (e.g., using surface electrodes instead of needle electrodes) materials may not be an impediment, as well as that the final system can be easily transported, mounted and deployed.

## SUMMARY OF THE INVENTION

In order to mitigate the shortcomings mentioned above, the present invention proposes devices and methods for reducing the noise arising from the common mode component in biopotential acquisition recordings, which is tightly linked to two factors: a poor isolation of the acquisition system, and a significant unbalance between the impedances of the electrodes used for the acquisition. Thereby, the common mode component can be estimated and removed or reduced, thus allowing a clean, precise acquisition of a biopotential signal in an unexpensive and non-invasive manner, by using the device and/or by performing the method of the invention.

Advantageously, a first object of the invention relates to a device for reducing common mode noise in biopotential acquisitions, comprising:

- a first electrode and a second electrode;
- a first differential amplifier connected to the first and second electrodes;
- a third electrode connected to an effective ground;
- averaging means connected to the first and second electrodes (this may be, for instance, two equal-value, low-tolerance resistors, one connected to the first electrode and the other one connected to the second electrode or two voltage-follower active circuits, one connected to the first electrode and the other to the second electrode, and an analog summing amplifier for adding the outputs of both voltage followers, among others);
- a second differential amplifier connected to the third electrode and to the averaging means;
- means for combining a first output signal coming from the first differential amplifier with a second output signal coming from the second differential amplifier, the second output signal being weighted by a gain factor;
- gain factor control means connected to the means for combining the first and second output signals adapted to adjust the value and/or sign of the gain factor; and
- a registration unit connected to the means for combining the first and second output signals.

By using this device, a second object of the invention refers to a method for reducing common mode noise in biopotential acquisitions, comprising the following steps:

a) deploying a first electrode and a second electrode in the surroundings of a biopotential generation region;
b) obtaining, using a first differential amplifier, a first output signal by using as inputs the signal received by the first electrode and the signal received by the second electrode;
c) connecting a third electrode to an effective ground;
d) obtaining, using a second differential amplifier, a second output signal by using as inputs the signal received by the third electrode and an average of the signals received by the first and second electrodes;
e) combining the first output signal with the second output signal, the second output signal being weighted with a gain factor;
f) adjusting the gain factor such that the common-mode signal is minimised; and

g) registering the final combination of the first and second output signals once the gain factor has been adjusted.

As previously mentioned, a major benefit of using this device and method is the reduction of the common-mode signal which acts as noise in the acquisition of biopotentials, especially in those with low amplitudes such as (auditory) evoked potentials. The standard procedures known in the art do not estimate the common mode signal (second output signal), so, by means of said procedures, the elimination of the common mode component is inefficient. In some cases, these procedures include a third electrode set to ground like in the method of the invention. Nevertheless, this active ground is used to inject into the experimental subject a voltage through a very large resistor (of the order of 1 MOhm) that compensates the common mode (through an analog feedback), but with a very large time constant (i.e., very low frequencies, actually DC and slow fluctuations of the DC level) that in practice serves just to prevent the DC drift from saturating the amplifier, but not for specifically eliminating the common mode component. In the context of the invention, an effective ground is understood as a region of the subject under study, which is far from the biopotential generation region, i.e., a region of the subject which is far from the region in which the first and second electrodes are placed. Specific examples of different configurations regarding the placement of the first, second, and third electrodes will be described in subsequent parts of this document.

Using the device and method of the invention allows for acquiring very good quality biopotentials even using non-expensive, non-invasive materials and in circumstances in which the subject or setting is not electrically isolated. Surface electrodes can be efficiently used even if having very unbalanced contact impedances since the method of the invention serves to minimize the common mode noise arising from said unbalance. In this way, needle electrodes, with typically smaller unbalances but more expensive and more invasive, do not need to be used. It is to be noted that the method can be used with said needle electrodes, if needed.

On the other hand, interference or noise coming from external sources, such as the mains, is also reduced by implementing the method of the invention, so that using isolation rooms is not essential in order to acquire the biopotentials of interest with good quality. This reduces costs and allows for a more convenient acquisition process, since the subject does not need to be transferred to a specific location. It is to be noted that the method can be used with isolation rooms, if needed.

In another embodiment of the invention, the device further comprises monitoring means such as a speaker, a set of speakers, a set of headphones and/or a VU meter, connected to the means for combining the first and second output signals. This allows for the combination of the first and second output signals to be performed analogically and outputted to the monitoring means, such that the gain factor can be adjusted manually by a user monitoring the outputted signal. In this case, a speaker, a set of headphones, a VU meter and/or other monitoring instruments that allow for listening and/or for inspecting visually the combined signal may be used, as stated. Once the combined signal is outputted to one or more of these monitoring instruments, a user may vary the gain factor until the sound or reading in said instrument/s is the optimal one. Since the biopotential signal and the common mode noise are statistically independent processes, the optimal value for the gain factor is the one which minimizes the power of the combined signal. This can be tracked with the monitoring means while varying the gain factor.

In another embodiment of the invention, the device further comprises:

- a first analog-to-digital converter connected to the output of the first differential amplifier, adapted to digitize the first output signal;
- a second analog-to-digital converter connected to the output of the second differential amplifier, adapted to digitize the second output signal; and
- processing means connected to the first and second analog-to-digital converters, adapted to automatically adjust the value and/or sign of the gain factor. This allows for the combination of the signals and the adjustment of the gain factor to be done automatically instead of analogically and manually. Referring to an embodiment of the method of the invention, between steps d) and e), the following steps are performed:

    d') digitizing the first and second output signals by using the first and second analog-to-digital converters, and sending the digitized first and second output signals to the processing means; and
    d") adjusting the gain factor automatically using the processing means by minimizing the energy of the combination of the first and second output signals.

In the case in which the processing means and the first and second analog-to-digital converters are available for use, this embodiment represents a more accurate way of reducing the common mode noise in biopotential acquisition than the previous ones (cheaper and more interesting when numerical processing means and/or analog-to-digital converters are not available). Whereas the manual regulation of the gain factor according to the output in the monitoring instruments is subjective and depends on the skills of the user, digitizing the first and second output signals and processing them numerically is more precise. The processing means may be a computer with instructions that, when executed, carry out the minimization of the energy of the combined signal, deriving the appropriate gain factor that maximally reduces the common mode noise. This optimization of the gain factor is therefore done automatically in a statistical framework. It has been experimentally proven that the value of the gain factor is very stable since it fluctuates little in biopotential acquisition procedures for auditory evoked potentials in long duration tests of more than 20 minutes.

Common mode noise reduction can be improved depending on the characteristics of this noise and how it affects the recorded signals. For instance, in another embodiment of the invention, the combination of the first and second output signals is performed independently for different frequency bands and the gain factor is adjusted for each frequency band independently. This allows for removing common mode noise coming from specific sources with well determined frequencies (such as the interference of the mains' signal) more accurately. In another embodiment of the invention, the gain factor, instead of being one or more constants, is time-dependent, and the combination of the first and second output signals as well as the adjustment of the gain factor is performed dynamically. This is advantageous in the cases in which, for example the contact impedances of the electrodes vary slowly throughout the biopotential acquisition procedure. Another embodiment of the invention relates to the adjustment of the gain factor using linear predictive coding based on upstream - downstream samples up to a certain length.

In another embodiment of the invention, before step a), at least one stimulus is delivered to a subject to induce a bioelectrical response. For instance, a pattern of audio signals can be delivered to a subject using a speaker or a set of headphones so that an auditory evoked potential is generated and then acquired by implementing the previously described method. Alternatively, visual stimuli such as light signals can be delivered, among others. Studies of the synchronization of the stimulus/stimuli and their corresponding response can be performed more accurately since the method provides a cleaner biopotential signal. For this, an embodiment relating to the device of the invention further includes:

- at least one digital-to-analog converter connected to the processing means; and
- a stimulus delivering unit connected to the digital-to-analog converter.

The stimulus delivering unit may be an auditive stimulus delivering unit comprising at least one of the following: a speaker, a set of speakers and/or a set of headphones; and/ or a visual stimulus delivering unit comprising at least one of the following: a controlled illumination device, a light and/or video display and/or a projection system.

In another embodiment of the invention, the device is designed to be modular. Specifically, the first and second differential amplifiers and a selection of at least one of the following elements:

- averaging means connected to the first and second electrodes;
- means for combining the first and second output signals, the second output signal being weighted by a gain factor;
- gain factor control means to adjust the value and/or sign of the gain factor; and/or
- monitoring means connected to the means for combining the first and second output signals,

may be comprised in a modular stage, called in the following an intermediate interface, which is detachable.

This intermediate interface reduces the noise compared to other configurations by providing electromagnetic shielding for the analog electronics previous to the amplification with the differential amplifiers. Moreover, it reduces the number of elements, avoiding several wiring connections. Technically, the working environment will be cleaner, with fewer elements, with less chance of making a mistake when assembling it.

The intermediate interface can be implemented in three different versions: one with two outputs (first and second output signals), one with one output (combined signal), or one with three outputs (first, second and combined signals). This last version would give flexibility in terms of possible configurations depending on the availability of processing means and analog-to-digital converters and the requirements of the process and of the rest of the equipment used.

On the other hand, and depending on the characteristics of the signal to be recorded (mainly its bandwidth), an audio interface or an audio mixing console could be used as a digitizing system, in which case the intermediate interface could be designed so that the power supply of the differential amplifiers is provided by 48 V DC "phantom" power, available in most mixing consoles and audio interfaces.

In summary, the proposed device and method allow for the acquisition of good quality biopotential signals with very reduced common mode noise in a non-expensive, non-invasive, modular and flexible way.

## BRIEF DESCRIPTION OF DRAWINGS

FIG. 1 shows a schematic representation of an electric circuit corresponding to the device of the invention, according to a first preferred embodiment thereof. Under this embodiment, the circuit is designed so that the gain factor can be adjusted manually, by inspecting the combination of the signals, for example visually or aurally.

FIG. 2 shows a schematic representation of an electric circuit corresponding to the device of the invention, according to a second preferred embodiment thereof. Under this embodiment, the circuit is designed so that the adjustment of the gain factor is performed automatically, for example by processing means such as a computer.

FIG. 3 shows a schematic representation of a device of the invention according to another embodiment of the invention, as well as an exemplary deployment of said device for reducing common mode noise in biopotential acquisition processes for recording auditory evoked responses.

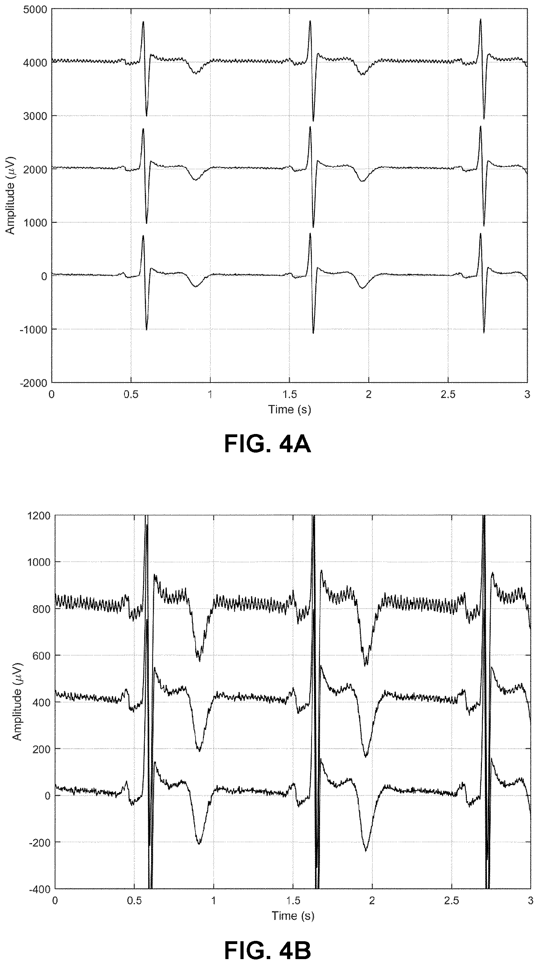

FIG. 4 shows the amplitude of a same ECG acquired by a prior-art procedure (upper curve), by following the proposed method in a basic implementation with one parameter to reduce the common mode noise (centre curve) according to one preferred embodiment thereof and by following the proposed method in an advanced implementation with several parameters to reduce the common mode noise (lower curve) according to another preferred embodiment thereof, expressed as function of the time of recording. FIG. 4A shows the full range of the results while FIG. 4B shows a detailed view of the results presented in FIG. 4A.

FIG. 5 shows the power spectral density for an ECG of 25 seconds, corresponding to a prior-art acquisition procedure.

FIG. 6 shows the power spectral density for an ECG of 25 seconds, corresponding to the method of the invention in a basic implementation according to a preferred embodiment thereof.

FIG. 7 shows the power spectral density for an ECG of 25 seconds, corresponding to the method of the invention in an advanced implementation according to another preferred embodiment thereof.

FIG. 8 shows the amplitude of a same ECG, recorded under high level of common mode noise, acquired by a prior-art procedure (upper curve), by following the proposed method in a basic implementation (centre curve) according to one preferred embodiment thereof and by following the proposed method in an advanced implementation (lower curve) according to another preferred embodiment thereof, expressed as function of the time of recording. FIG. 8A shows the full range of the results while FIG. 8B shows a detailed view of the results presented in FIG. 8A.

FIG. 9 shows the power spectral density for an ECG of 25 seconds recorded under high levels of common mode noise, corresponding to a prior-art acquisition procedure.

FIG. 10 shows the power spectral density for an ECG of 25 seconds recorded under high levels of common mode noise, corresponding to the method of the invention in a basic implementation according to a preferred embodiment thereof.

FIG. 11 shows the power spectral density for an ECG of 25 seconds recorded under high levels of common mode noise, corresponding to the method of the invention in an advanced implementation according to another preferred embodiment thereof.

Fig. 12 shows the amplitude of a same EEG acquired by a prior-art procedure (upper curve), by following the proposed method in a basic implementation with one parameter to reduce the common mode noise (centre curve) according to one preferred embodiment thereof and by following the proposed method in an advanced implementation with several parameters to reduce the common mode noise (lower curve) according to another preferred embodiment thereof, expressed as function of the time of recording.

FIG. 13 shows the power spectral density for an EEG of 420 seconds, corresponding to a prior-art acquisition procedure.

FIG. 14 shows the power spectral density for an EEG of 420 seconds, corresponding to the method of the invention in a basic implementation according to a preferred embodiment thereof.

FIG. 15 shows the power spectral density for an EEG of 420 seconds, corresponding to the method of the invention in an advanced implementation according to another preferred embodiment thereof.

FIG. 16 shows a set of deconvolved auditory brainstem responses (a particular response evoked by auditory stimulation) resulting from biopotential acquisition obtained using the EEG corresponding to a prior-art procedure.

FIG. 17 shows a set of deconvolved auditory brainstem responses resulting from biopotential acquisition obtained using the EEG corresponding to the method of the invention in a basic implementation according to a preferred embodiment thereof.

FIG. 18 shows a set of deconvolved auditory brainstem responses resulting from biopotential acquisition obtained using the EEG corresponding to the method of the invention in an advanced implementation according to another preferred embodiment thereof.

## NUMERIC REFERENCES USED IN THE DRAWINGS

| (1, 1', 1") | First, second and third electrodes |
| --- | --- |
| (2) | Biopotential generation region |
| (3, 3') | First and second differential amplifiers |
| (4, 4') | First and second output signals |
| (5) | Averaging means |
| (6) | Combined output signal |
| (7) | Gain factor |
| (8) | Means to combine the first and second output signals |
| (9) | Final output signal |
| (10) | Registration unit |
| (11) | Gain factor control means |
| (12) | Monitoring means |
| (13, 13') | First and second analog-to-digital converters |
| (14) | Processing means |
| (15) | Intermediate interface |
| (16) | Conversion stage |
| (17) | Digital-to-analog converter |
| (18) | Stimulus delivering means |

## DETAILED DESCRIPTION OF THE INVENTION

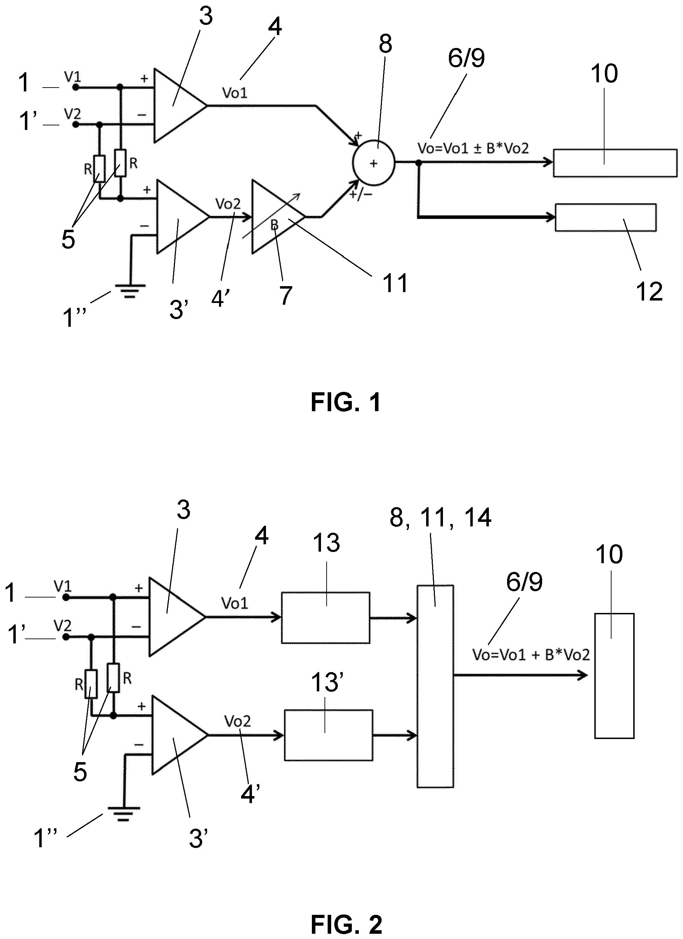

Several preferred embodiments of the invention, shown in Figs. 1-18, will be now described for illustrative, but not limiting purposes.

Under these embodiments, and according to Figs. 1-3, the invention is based on the reception of signals from three electrodes: a first and a second electrode (1, 1'), which are adapted for their placement on a biopotential generation region (2), and a third electrode (1") adapted as a ground connection. With this configuration, the device of the invention allows for performing the following steps:

- amplifying, with a first differential amplifier (3), the signal coming from the first and second electrodes (1, 1'), obtaining a first output signal (4);
- amplifying, with a second differential amplifier (3'), an estimate of the common mode signal, obtained by setting one of the inputs of the second differential amplifier (3') to ground (signal coming from the third electrode (1")) and the other input to an estimate of the average signal of the first and second electrodes (1, 1') (obtained with averaging means (5) that may be, for instance, two equal, low-tolerance resistors), obtaining a second output signal (4'); and
- combining the first and second output signals (4, 4') into a combined output signal (6), with the second output signal

7

(4') being weighted with a gain factor (7). The means to combine (8) the first and second output signals may be either analog electronics or a numerical procedure.

- adjusting the value and sign of the gain factor (7) such that the common-mode noise is minimised in the combined output signal (6).
- registering the final output signal (9), i.e., the combined output signal (6) once the gain factor (7) has been appropriately adjusted, using a registration unit (10).

More specifically, Fig. 1 shows a schematic representation of an electric circuit corresponding to a device according to an embodiment of the invention. Signals V1 and V2, coming from the first and second electrodes (1, 1') respectively, are inputted into the first differential amplifier (3), which outputs a first output signal (4) Vo1 corresponding to the differential signal of the active electrodes (first and second electrodes (1, 1')) polluted with a common-mode noise component due to the electrodes' impedance unbalance. On the other hand, the signal coming from the third electrode (1") (ground signal) and the average of the signals V1 and V2 are inputted to the second differential amplifier (3'). The average is obtained by using averaging means (5) such that, for instance, by connecting V1 to a resistor, V2 to another resistor of equal value, and then connecting both resistors with one another. The output of this second differential amplifier, Vo2, corresponding to a second output signal (4'), thus contains exclusively a common mode signal which, for the biopotential acquisition, acts just as noise.

The circuit also includes gain factor control means (11) for the second output signal (4') (common mode signal, Vo2) adapted to modify the value and sign of said gain factor (7). The gain factor control means (11) may comprise a switch to control the sign and a potentiometer to control the value of the gain factor (7). Then, when the first and second output signals (4, 4') are combined, the combined output signal (6) reads Vo = Vo1 + B * Vo2. Since Vo1 is polluted with common mode noise, and, therefore, can be decomposed as Vo1= Vdiff + C*Vo2, with Vdiff being the clean, pure differential signal of V1 and V2, Vo2 being the common mode signal, and C a parameter controlling the amount of common mode signal in Vo1, adjusting the value and sign of the gain factor (7), B, allows for eliminating (or at least reducing) the common mode signal present in Vo1. Once the optimal value of the gain factor (7) is found, the combined output signal (6) of the first and second output signals (4, 4'), is registered or recorded using a registration unit (10), i.e. an analog or digital registration system appropriate for the bandwidth of the biopotentials to be registered (for instance, an audio recorder if the bandwidth of the biopotentials is compatible with the audio bandwidth).

In a preferred embodiment of the invention, the gain factor (7) can be adjusted manually. In this embodiment, the combined output signal (6) is outputted to monitoring means (12) such as a set of headphones through which the user listens. To determine the optimum gain factor (7) value and sign, i.e., the one that minimizes the presence of the common mode signal, and therefore optimizes the signal-to-noise ratio (SNR) in the resulting final output signal (9), the user varies the value and sign of the gain factor (7) by using the gain factor control means (11) until the power of the combined output signal (6) as displayed in the monitoring means (12) is minimized. This process can be performed since the differential signal and the noise are statistically independent (and therefore uncorrelated) signals.

In other preferred embodiments of the invention, the monitoring means (12) correspond to a speaker or a set of speakers, a VU meter, or other means for visualizing and/or listening to the signal.

Fig. 2 shows another preferred embodiment of the invention in which, instead of using a fully analogic circuit as the one depicted in Fig. 1, the first and second output signals (4, 4') Vo1 and Vo2 are digitized by a first and second analog-to-digital converters (13, 13'), respectively and then processed numerically. It is to be noted that either a two-channel analog-to-digital converter or two single-channel analog-to-digital converters may be used. In any of such cases, the conversion must be synchronous in order not to lose the coherence between the signals. This results in a more precise determination of the gain factor (7) value and sign that minimizes the common mode signal of the combined output signal (6). In this embodiment, a computer (or alternatively a microcontroller, or a programmable digital-signal-processor) is used as processing means (14). This computer comprises, at the same time, the means to combine (8) the first and second output signals (4, 4'), and the gain factor control means (11) for obtaining the final output signal (9).

More specifically, the combination of the digitized first and second output signals (4, 4') with the second output signal (4') being weighted with a gain factor (7), and the optimization of said gain factor (7) in order to eliminate the common mode signal, are performed numerically using processing means (14), such as a computer. As stated before, since the differential signal and common-mode signal are statistically independent processes, and since the second output signal (4'), Vo2, contains only common-mode signal, the SNR becomes maximal when the energy of the combined output signal (6), Vo, is minimized, i.e., when the B*Vo2 term cancels (or at least maximally compensates) the common-mode signal content present in Vo1. Assuming zero mean signals (no DC content):

$$E[Vo^2] = E[(Vo1 + B*Vo2)^2] = E[Vo1^2] + E[(B*Vo2)^2] + 2\,E[B*Vo1*Vo2], \qquad (1)$$

where E[x] is the expected value of x. The expected value $E[Vo^2]$ is minimized for the value of the gain factor (7), B, that makes the derivative of E[Vo^2] with respect to the gain factor (7), B, zero. This corresponds to B = - E[Vo1*Vo2] / E[Vo2*Vo2], i.e., the correlation of both signals normalized with the variance of the common mode signal, changed sign. This relation is used by the numerical procedure in order to optimize the value and sign of the gain factor (7) that maximally eliminates the common mode signal in the combined output signal (6) in a very precise way. Once this is performed, the resulting final output signal (9), i.e., the combined output signal (6) once the gain factor (7) has been adjusted, is registered and stored in a digital memory unit.

From the procedure described above, common mode noise reduction can be further improved depending on the characteristics of this noise and how it affects the recorded signals. In this regard, the following embodiments are disclosed:

- Instead of adjusting only one gain factor (7) independently of the frequency of the first and second output signals (4, 4'), these latter signals are segmented into different frequency bands and an optimal gain factor (7) is determined or adjusted for each of these bands.
- Instead of adjusting a constant value of the gain factor (7), a time-dependent gain factor (7) can be employed. Thus, the adjustment can be performed dynamically, allowing for the gain factor (7) to vary slowly with time, which provides an extra advantage, for example, if the electrodes' impedances vary during the acquisition process.
- Finally, a standard, linear predictive coding approach can be employed in order to adjust several values for the gain factor (7), based on upstream - downstream samples up to a certain length. Under this approach, the output signal is modelled as:

$$Vo(n) = Vo1(n) + B(-K)\ Vo2(n-K) + B(-K+1)\ Vo2(n-K+1)+\ldots$$

$$\ldots + B(0)\ Vo2(n) + B(1)\ Vo2(n+1) + \ldots + B(K)\ Vo2(n+K) \qquad (2)$$

and the optimal combination of Vo1 and Vo2 is performed by minimizing the expected value E[Vo^2] with respect to all the gain factors B(k), i.e. obtaining a system of 2K+1 linear equations with 2K+1 unknowns (that can be easily represented under matrix representation and resolved by matrix inversion).

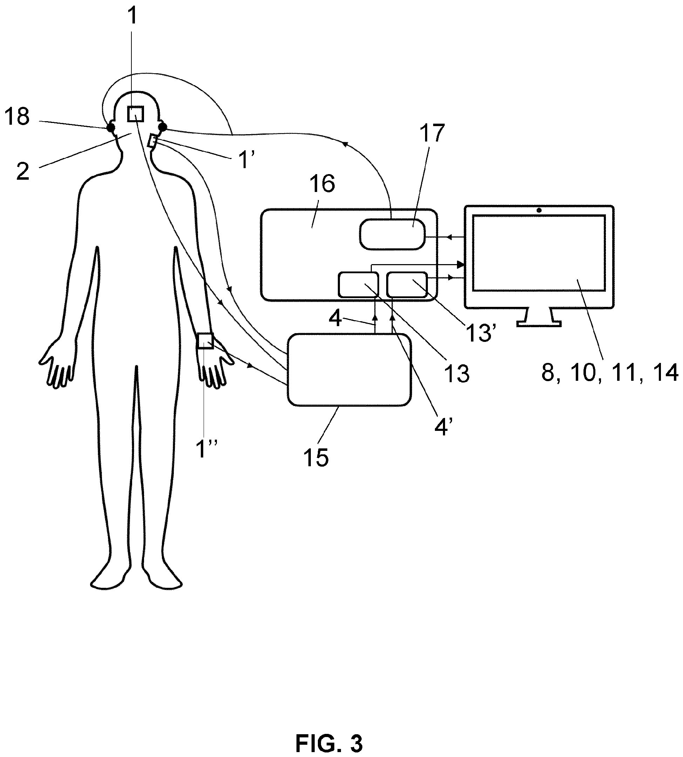

Fig. 3 shows a schematic representation of a system including the device of the invention and a possible deployment, the device comprising, first, at least three electrodes: a first (active) and second (reference) electrodes (1, 1') placed nearby a biopotential generation region (2), and a third electrode (1") (ground) placed far from said biopotential generation region (2). In FIG. 3, the first electrode (1) is placed in the forehead, the second electrode (1') is placed in a mastoid region, and the third electrode (1") is placed in the wrist of a subject, with the aim of acquiring auditory evoked potentials. If, for instance, an electrocardiogram was to be performed, the first and second electrodes (1, 1') would be placed close to the heart (mainly in the chest), and the third electrode (1") would be placed in an ankle.

The device also comprises an intermediate interface (15), acting as a modular stage that is detachable from the rest of the system, configured to obtain, from the signals received by the electrodes (1, 1', 1"), a differential signal from the first and second electrode signals and a common mode signal estimate from the average of the first and second electrode signals and the third electrode signal (ground). This would correspond to the first part of the circuits shown in Figs. 1 and 2, in which, by using two differential amplifiers (3, 3'), the first and second output signals (4, 4'), Vo1 and Vo2, are obtained. The first and second output signals (4, 4') are outputted from the intermediate interface (15).

In some embodiments of the invention, the intermediate interface (15) comprises means to combine (8) the first and second output signals (4, 4'), the second output signal (4') being weighted by a gain factor (7). In this case, the intermediate interface (15) comprises electronic circuitry allowing for this combination to be performed as well as gain factor control means (11). Furthermore, it may also comprise an output corresponding to the combined output signal (6). This output allows for monitoring means (12) (a speaker, a set of headphones, a VU meter, and/or other instruments) to be connected to the intermediate interface (15) such that a user can inspect the combined output signal (6) visually or aurally in order to manually achieve the appropriate gain factor (7).

The registration and storage of the final output signal (9) can be performed using a conversion stage (16) (such as analog-to-digital/digital-to-analog (AD/DA) conversion means) comprised by the device, connected to the intermediate interface (15) and to the registration unit (10) (for instance a digital memory or a computer). The conversion stage (16), that may be for instance an appropriate AD/DA acquisition board, (or in the case of biopotentials compatible with the audio bandwidth, a commercial digital audio interface, a digital audio mixing console or a sound card), comprises at least a single input for the combined output signal (6), at least one analog-to-digital converter and at least one digital output for the digitized combined output signal (6) or final output signal (9).

However, another complementary possibility is to perform the combination of the first and second output signals (4, 4') using the conversion stage (16) and the processing means (14). The conversion stage (16), that may be, for instance, an appropriate AD/DA acquisition board, (or in the case of biopotentials compatible with the audio bandwidth, a commercial digital audio interface, a digital audio mixing console or a sound card), comprises in this case at least two inputs, two analog-to-digital converters, and two digital outputs (one for each of the first and second output signals (4, 4') provided by the intermediate interface (15)). The processing means (14) receive the digitized first and second output signals (4, 4'), perform the combination adjusting the gain factor (7) numerically as described above to obtain the final output signal (9) and provide said final output signal (9) to the registration unit (10), also comprised by the device. In the case in which the processing means (14) correspond to a computer, it can also be used to register or store the final output signal (9) once the gain factor (7) has been conveniently adjusted, although, as mentioned before, other registration or storage units (8), such as an appropriate analog or digital recorder can be used instead by connecting them directly to the output providing the final output signal (9).

The processing means (14) can also be used to configure one or more stimuli or sets of stimuli to be delivered to a subject, for instance for the acquisition of auditory evoked potentials. In this configuration, the stimulus or stimuli are determined using the processing means (14), sent to the conversion stage (16) (further comprising in this embodiment a digital-to-analog converter (15)), converted to an audio signal and then delivered to the subject through stimulus delivering means (16), such as a set of headphones connected to the conversion stage (16). This stimuli or sets of stimuli must be synchronized with the previously received signals for a precise processing of the resulting acquired biopotentials.

In all the embodiments concerning the device of the invention, the connection between the electrodes (1, 1', 1") and the intermediate interface (15) and between the intermediate interface (15) and the conversion stage (16) are done using balanced audio cables, although other wire or cables may be used.

Examples of application:

In the following, two examples of application of the method by means of a device as described above are presented in order to demonstrate the effectiveness of said method and device in reducing the common mode noise component in a biopotential acquisition process, in the particular case of acquisition of electrocardiograms (first example) and auditory evoked potentials corresponding to auditory brainstem responses (second example).

A device as described above was built using the following components:

- three adhesive, disposable electrodes (1, 1', 1");
- one balanced audio cable with three crocodile connectors in one end (to connect to the electrodes) and one 3-pin XLR connector in the other end (to connect to the intermediate interface (15));
- an intermediate interface (15) comprising electronic circuitry (at least a first and second differential amplifiers (3, 3'), resistors, etc.);
- a commercial digital audio interface acting as conversion stage (16) with a bandwidth in the range of 1 Hz - 20 kHz;
- two standard balanced audio cables with XLR connectors in both ends, to insert the first and second output signals (4, 4') from the intermediate interface (15) to the digital audio interface;
- a computer, connected to the digital audio interface using a USB connection, acting as processing means (14); and
- two sets of headphones, one of them acting as monitoring means (12) and the other acting as stimulus delivering means (18), the last used for auditory stimulation of the subject under exploration in the second example (acquisition of auditory evoked potentials);

With these elements, several tests were performed. Different configurations of the device were tested, and different sets of stimuli were delivered to a subject in the second example. For instance, settings as displayed in Figs. 1 and 2 (manual vs numerical adjustment of the gain factor (7)) were explored.

In the approach shown in FIG. 1, it was clearly perceived by the user how there was a value of the gain factor (7), either with positive or negative sign (depending on the experiment performed), for which the average combined output signal (6) level became minimum, appreciating an increase of the signal level both if the value of the gain factor (7) was increased or reduced with respect to this optimum value. Estimation of the optimum value of the gain factor (7) with an accuracy of the order of 1 dB was, nevertheless, difficult, since this technique is based on a subjective assessment of a signal containing noise.

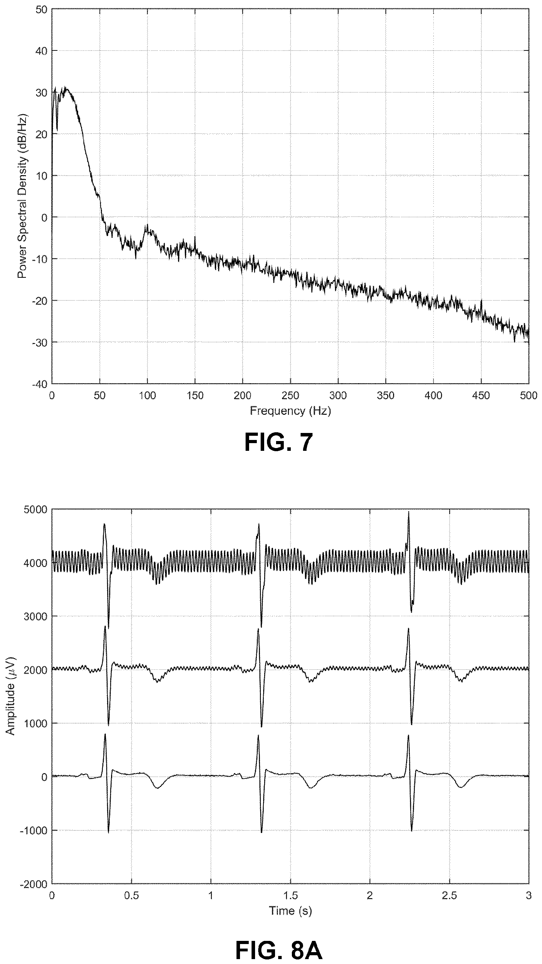

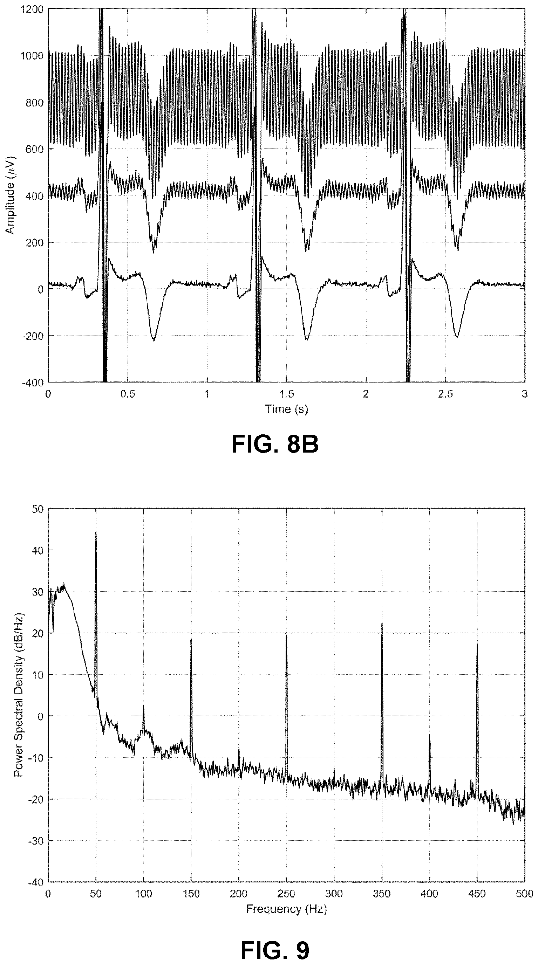

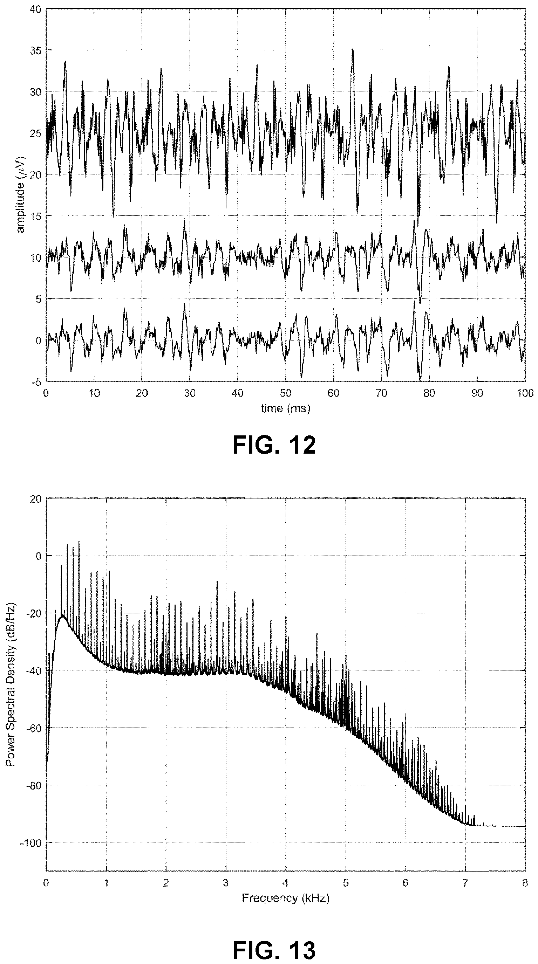

In the approach depicted in FIG. 2 (digitizing the first and second output signals (4, 4'), Vo1 and Vo2, and obtaining the optimal value of the gain factor (7) numerically in a statistical framework), the acquisition of a biopotential by the standard procedure, i.e., registering only the first output signal (4), could be compared with the proposed method of the invention. The experiments performed correspond to the acquisition of ECG, under favourable common mode noise condition (FIGs. 4-7), acquisition of ECG under unfavourable common mode noise condition (FIGs. 8-11), and acquisition of EEG for registering auditory evoked potentials with click-type stimuli of different intensities (FIGs. 12-18).

For the acquisition of ECG, the active and reference electrodes, i.e., the first and second electrodes (1,1'), were placed on the chest, in the precordial positions V4 and Vr4, respectively, and the ground electrode, i.e., the third electrode (1"), in the right wrist. The duration of the ECG recording was 25 seconds. In order to generate the unfavourable common mode noise condition, the subject under exploration was asked to touch the floor with one foot, which causes some current drift associated to the electromagnetic interference and increases the common mode noise voltage in the recording electrodes.

In FIG. 4, the amplitudes of the same ECG acquired by prior-art procedure (upper curve), by following the method of the invention in a basic implementation (centre curve) and by following the method of the invention in an advanced implementation (lower curve) are displayed. Three seconds of ECG are represented in FIG.4, where three heart pulses can be observed. While in FIG. 4A the complete vertical range of the waveform is displayed, in FIG. 4B a detail of the ECG is displayed in order to allow the observation of the noise affecting the waveform. For the prior-art procedure (upper curve), only the first output signal (4) Vo1 is used. The basic implementation of the method of the invention (centre curve) was performed according to the minimization of equation (1), i.e. by optimizing the gain factor (7) in the combined output signal (6). The advanced implementation of the method of the invention applied in this example (lower curve) was performed using three frequency bands (0-60 Hz, 60-120 Hz, 120-500 Hz) and applying the combination for each of these frequency bands using upstream-downstream samples of the common mode estimation up to 4 samples (at a sampling rate of 1 kHz), according to a linear predictive model as described in equation (2). Therefore, this advanced implementation involves the estimation of 9 parameters for each frequency band, with a total of 27 parameters. In the waveform corresponding to the prior-art acquisition, some noise can be observed, particularly evident in FIG. 4B, and mostly corresponding to an oscillation of 50 Hz (which can be associated with noise from the electrical power supply network). The common mode noise is significantly reduced with the acquisition based on the method of the invention in the basic implementation. The method of the invention in the advanced implementation provides an additional reduction of the common mode noise. The amplitude of the ECG is 153.7 $\mu$V rms, and the amplitude of the noise (estimated using the portion of ECG in the time interval between 1.1 s and 1.4 s) is 17.4, 11.2 and 10.6 $\mu$V rms for the acquisitions with the prior-art method, the method of the invention in the basic implementation and the method of the invention in the advanced implementation, respectively. According to these estimations, the SNR of the ECGs acquired with these methods is, respectively, 18.9, 22.7 and 23.3 dB, which illustrates the improvement provided by the method of the invention in both the basic and the advanced implementation.

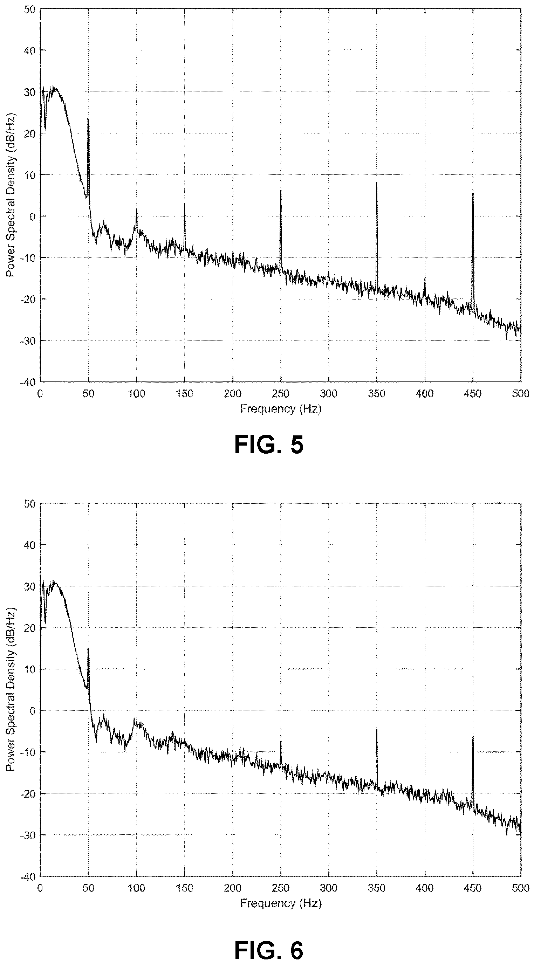

The plots presented in FIGs. 5-7 show the power spectral density for an ECG of 25 seconds of duration, corresponding to the prior-art procedure (FIG. 5), the method of the invention under the basic implementation (FIG. 6), and the method of the invention under the advanced implementation (FIG. 7). In Fig. 5, several harmonics of 50 Hz (spectral peaks at frequencies multiple of 50 Hz), which can be associated with noise from the electrical power supply network, are visible. These harmonics are significantly attenuated (more than 10 dB) by applying the method of the invention in the basic implementation (FIG. 6) and have been virtually eliminated by the application of the method of the invention in the advanced implementation (as observed in FIG. 7).

FIG. 8 shows similar plots to those presented in FIG. 4, corresponding to the same subject and the same acquisition session, where the subject under exploration was asked to put one foot on the floor (i.e. without any isolation element), causing a low current drift to ground that artificially increases the common mode noise associated to the interference of the electrical power supply network, with the aim of testing the method of the invention under unfavourable noise conditions. As in FIG. 4, in this figure the amplitudes of the ECG acquired with the prior-art procedure (upper curve), with the method of the invention in the basic implementation (centre curve) and the method of the invention in the advanced implementation (lower curve) are displayed with the complete vertical range (FIG. 8A) and with a detailed representation (FIG. 8B). The unfavourable common mode noise conditions are evident in the ECG acquired with the prior-art procedure, with a very strong oscillation of 50 Hz of about 400 $\mu$V peak-to-peak. The method of the invention in the basic implementation significantly reduces this oscillation, even though some remaining noise is present. The method of the invention in the advanced implementation provides a better reduction of the common mode noise. In these recordings, the amplitude of the ECG is 154.4 $\mu$V rms, and the amplitude of the noise (estimated using the portion of ECG in the time interval between 1.78 s and 2.08 s) is 140.7, 18.6 and 6.9 $\mu$V rms for the acquisitions with the prior-art method, the method of the invention in the basic implementation, and the method of the invention in the advanced implementation, respectively. According to these estimations, the SNR of the ECGs acquired with these methods is, respectively, 0.8, 18.4 and 27.0 dB, which illustrates the improvement provided by the method of the invention in both the basic and the advanced implementation, particularly under high common mode noise conditions.

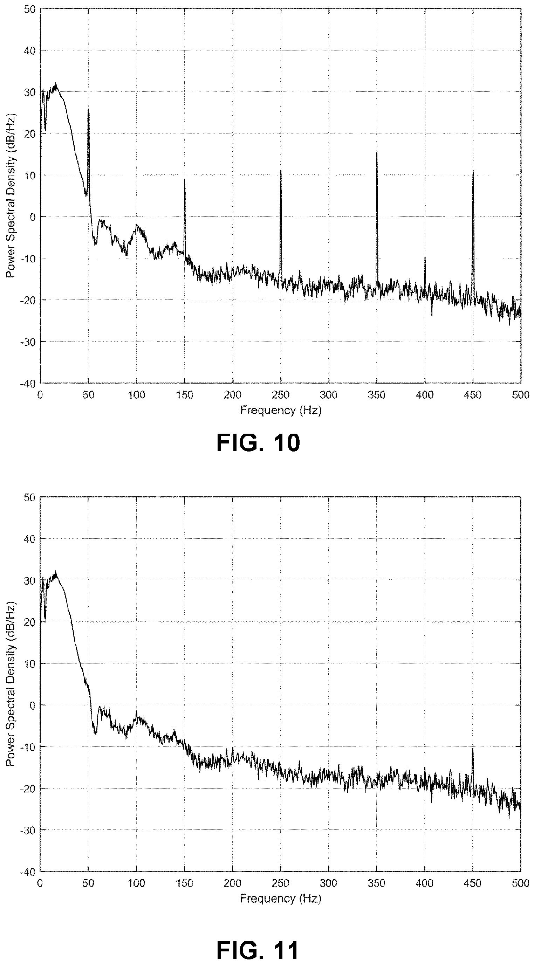

The plots displayed in FIGs. 9-11 show the power spectral density for an ECG of 25 seconds of duration, corresponding to the prior-art procedure (FIG. 9), the method of the invention under the basic implementation (FIG. 10), and the method of the invention under the advanced implementation (FIG. 11), when the ECG was acquired under unfavourable noise conditions. In FIG. 9, several large harmonics of 50 Hz, are visible. These harmonics are significantly attenuated thanks to the application of the method of the invention in the basic implementation (FIG. 10) and have been virtually eliminated by the application of the method of the invention in the advanced implementation (as observed in FIG. 11).

For the acquisition of auditory brainstem responses, the active and reference electrodes, i.e., the first and second electrodes (1,1'), were placed on the forehead and the right mastoid, respectively, and the ground electrode, i.e., the third electrode (1"), in the right wrist. The evoked responses were elicited with rarefaction clicks of 0.1 ms, with different levels between 20 dB and 80 dB (normal hearing level) in steps of 10 dB. The duration of the EEG recording was 420 seconds (7 minutes) and the responses were deconvolved for each stimulation level, where each deconvolved response (at each stimulation level) results from the contribution of 2000 stimuli. The deconvolution for the estimation of the auditory brainstem responses has been applied to the EEG acquired by the prior-art procedure, to the EEG acquired by following the method of the invention in a basic implementation and by following the method of the invention in an advanced implementation. For the prior-art procedure, only the first output signal (4) Vo1 is used. The basic implementation of the method of the invention was performed according to the minimization of equation (1), i.e., by optimizing the gain factor (7) in the combined output signal (6). The advanced implementation of the method of the invention applied in this example was performed using upstream-downstream samples of the common mode estimation up to 12 samples (at a sampling rate of 16 kHz), according to a linear predictive model as described in equation (2), involving the estimation of 25 parameters in the reduction of the common mode noise.

In FIG. 12 the amplitudes of the same EEG acquired by a prior-art procedure (upper curve), by following the method of the invention in the basic implementation (centre curve) and by following the method of the invention in the advanced implementation (lower curve) are displayed. It is to be noted that only a 200 ms portion of EEG is shown to appreciate the conventional first output signal (4), and the final output signal (9) under the basic and advanced implementation of the method of the invention. As it can be observed, in all the three cases, the EEG is strongly affected by the noise (since the amplitudes of the auditory brainstem responses are typically around 0.5 $\mu$V peak-to-peak). This figure shows how, when applying the method of the invention, the amplitude of the processed signal decreases with respect to the prior-art procedure. Both implementations of the method of the invention provide EEGs with similar waveforms, with only slight differences. The amplitude of the processed signal decreases from 3.36 $\mu$V rms (prior-art) to 1.45 $\mu$V rms for the basic implementation and to 1.40 $\mu$V rms for the advanced implementation (which means an improvement of the signal-to-noise ratio of 7.32 dB and 7.60 dB, respectively).

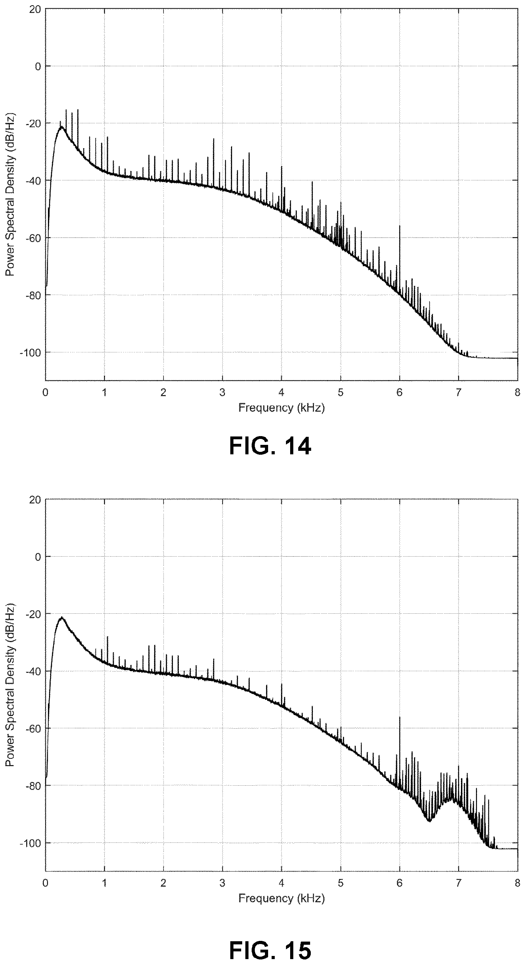

The plots presented in FIGs. 13-15 show the power spectral density for an EEG of 7 minutes of duration, corresponding to the prior-art (FIG. 13), the method of the invention in the basic implementation (FIG. 14) and the method of the invention in the advanced implementation (FIG. 15). In these figures a number of 50 Hz large harmonics (spectral peaks at frequencies multiple of 50 Hz), which can be associated with noise from the electrical power supply network, are visible. These harmonics are considerably attenuated (about 20 dB) by implementing the method of the invention. The advanced implementation of the method of the invention provides better attenuation of the harmonics than the basic implementation.

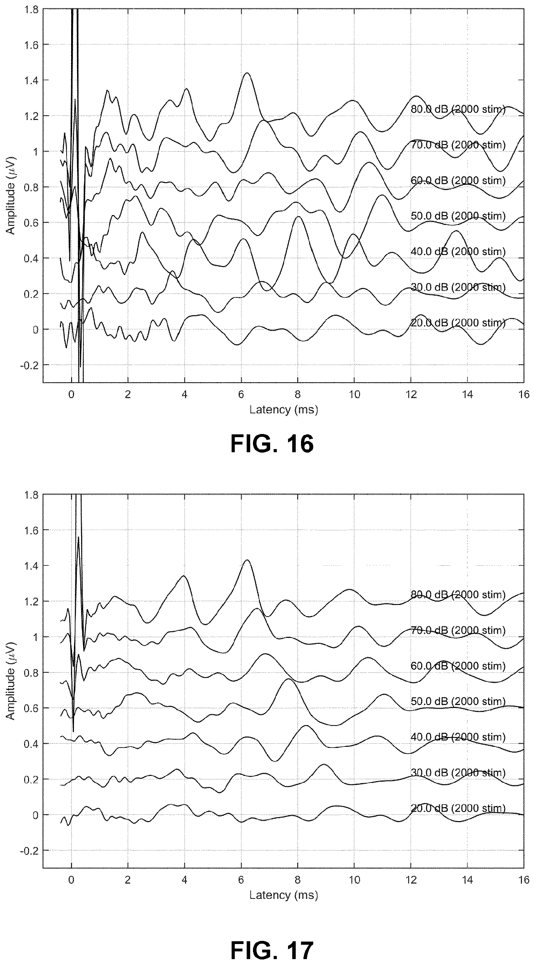

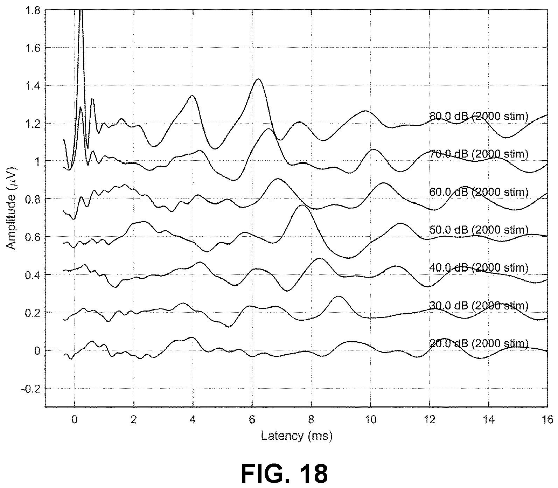

Finally, the plots displayed in FIGs. 16-18 show the deconvolved auditory brainstem responses (resulting from biopotential acquisition) obtained using the EEGs corresponding to the prior-art procedure (FIG. 16), the method of the invention in the basic implementation (FIG. 17) and the method of the invention in the advanced implementation (FIG. 18). In this figure, the evoked responses (each resulting from the contribution of 2000 stimuli) are observed with a very characteristic V-wave (peak whose latency increases from 6 ms for 80 dB stimuli to 9 ms at 20 dB). The proposed processing (with both the basic and the advanced implementations) significantly improves the quality of the responses (evoked potentials more consistent and less affected by noise). The quality of the evoked responses obtained with the basic and the advanced implementation is similar. However, the advanced implementation provides the responses with lower stimulation artifact (peaks in the latency interval 0-0.3 ms corresponding to an interference generated by the headphones transducer, with amplitude consistent with the stimulation level, i.e., increasing a factor 3.1 for each 10 dB increase in the stimulation level). The interference causing the stimulation artifact affects both electrodes and causes another common mode noise different than that caused by the electrical power supply network. The flexibility provided by the advanced implementation of the method of the invention contributes to an efficient reduction of both noise processes.

In all these cases the method of the invention reduced significantly the (common-mode) noise, improving the SNR of the acquired biopotentials.

**Claims**

1. Device for reducing common mode noise in biopotential acquisitions, **characterized in that** it comprises:

  - a first electrode (1) and a second electrode (1');
  - a first differential amplifier (3) connected to the first and second electrodes (1, 1');
  - a third electrode (1") connected to an effective ground;
  - averaging means (5) connected to the first and second electrodes (1, 1');
  - a second differential amplifier (3') connected to the third electrode (1") and to the averaging means (5);

- means for combining (8) a first output signal (4) coming from the first differential amplifier (3) with a second output signal (4') coming from the second differential amplifier (3'), the second output signal being weighted by a gain factor (7);
- gain factor control means (11) connected to the means for combining (8) the first and second output signals (4, 4') adapted to adjust the value and/or sign of the gain factor (7); and
- a registration unit (10) connected to the means for combining (8) the first and second output signals (4, 4').

2. Device according to the preceding claim, further comprising monitoring means (12) connected to the means for combining (8) the first and second output signals (4, 4'), wherein the monitoring means comprise at least one the following: a speaker, a set of speakers, a set of headphones or a VU meter.

3. Device according to any of the preceding claims, further comprising:

- a first analog-to-digital converter (13) connected to the output of the first differential amplifier (3) and a second analog-to-digital converter (13') connected to the output of the second differential amplifier (3'), the first and second analog-to-digital converters (13, 13') being adapted to digitize the first and second output signals (4, 4'); and
- processing means (14) connected to the first and second analog-to-digital converters (13, 13') adapted to automatically adjust the value and/or sign of the gain factor (7).

4. Device according to the preceding claim, further comprising:

- at least one digital-to-analog converter (17) connected to the processing means (14); and
- a stimulus delivering unit (18) connected to the digital-to-analog converter (17).

5. Device according to the preceding claim, wherein the stimulus delivering unit is an auditive stimulus delivering unit comprising at least one of the following: a speaker, a set of speakers or a set of headphones; and/ or a visual stimulus delivering unit comprising at least one of the following: a controlled illumination device, a light and/or video display or a projection system.

6. Device according to any of the preceding claims, wherein the first and second differential amplifiers (3, 3') and a selection of at least one of the following elements:

- averaging means (5) connected to the first and second electrodes (1, 1');
- means for combining (8) the first and second output signals (4, 4'), the second output signal being weighted by a gain factor (7);
- gain factor control means (11) to adjust the value and/or sign of the gain factor (7); and/or
- monitoring means (12) connected to the means for combining (8) the first and second output signals (4, 4'),

are comprised in an intermediate interface implemented as a modular stage which is detachable.

7. Method for reducing common mode noise in biopotential acquisitions, the method comprising the use of a device according to claim 1 and performing the following steps:

a) placing a first electrode (1) and a second electrode (1') in the surroundings of a biopotential generation region (2);
b) obtaining, using a first differential amplifier (3), a first output signal (4) from the signal received by the first electrode (1) and the signal received by the second electrode (1') as inputs;

and **characterized in that** the method further comprises the steps of:

c) connecting a third electrode (1") to an effective ground;
d) obtaining, using a second differential amplifier (3'), a second output signal (4') by using as inputs the signal received by the third electrode (1") and an average of the signals received by the first and second electrodes (1, 1'), obtained by using the averaging means (5);
e) combining the first output signal (4) with the second output signal (4') into a combined output signal (6), the second output signal (4') being weighted with a gain factor (7);
f) adjusting the gain factor (7) such that the common-mode in the combined output signal (6) is minimised and a final output signal (9) is obtained; and

g) registering the final output signal (9), once the gain factor (7) has been adjusted.

8. Method according to the preceding claim, wherein the device further comprises monitoring means (12) connected to the means for combining (8) the first and second output signals (4, 4'), the monitoring means comprising at least a speaker, a set of speakers, a set of headphones and/or a VU meter, and wherein the combination of the first and second output signals (4, 4') is performed analogically and outputted to the monitoring means (12) such that the gain factor (7) is adjusted manually by a user guided by the monitoring means' readings.

9. Method according to any of claims 7-8, wherein the final output signal (9) is digitized before performing step g).

10. Method according to any of claims 7-8, wherein the device further comprises:

- a first analog-to-digital converter (13) connected to the output of the first differential amplifier (3) and a second analog-to-digital converter (13') connected to the output of the second differential amplifier (3'), the first and second analog-to-digital converters (13, 13') being adapted to digitize the first and second output signals (4, 4'); and
- processing means (14) connected to the first and second analog-to-digital converters (13, 13') adapted to automatically adjust the value and/or sign of the gain factor (7); and

where said method further comprises performing, between steps d) and e), the steps:

d') digitizing the first and second output signals (4, 4') by using the at least one analog-to-digital converter (10), and sending the digitized first and second output signals (4, 4') to the processing means (14); and
d") adjusting the gain factor (7) automatically using the processing means (14) by minimizing the energy of the combination of the first and second output signals (4, 4') with respect to the gain factor applied to the second output signal (4').

11. Method according to the preceding claim, wherein the combination of the first and second output signals (4, 4') is performed independently for different frequency bands and wherein the gain factor (7) is adjusted independently for each frequency band.

12. Method according to any of claims 10-11, wherein the gain factor (7) is time-dependent and wherein the combination of the first and second output signals (4, 4') and the adjustment of the gain factor (7) is performed dynamically.

13. Method according to any of claims 10-12, wherein more than one gain factors (7) are adjusted using a linear predictive coding model.

14. Method according to any of the claims 10-13, wherein the device further comprises at least one digital-to-analog converter connected to the processing means and a stimulus delivering unit, and wherein before step a), at least one stimulus is delivered to a subject to evoke a bioelectrical response.

15. Method according to the preceding claim, wherein the stimulus delivering unit is an auditive stimulus delivering unit comprising at least one of the following: a speaker, a set of speakers or a set of headphones; and/ or a visual stimulus delivering unit comprising at least one of the following: a controlled illumination device, a light and/or video display and/or a projection system, and wherein the stimulus is an auditory stimulus or a visual stimulus.

**FIG. 1**

**FIG. 2**

**FIG. 3**

FIG. 4A

FIG. 4B

## FIG. 5

## FIG. 6

FIG. 7

FIG. 8A

FIG. 8B

FIG. 9

FIG. 10

FIG. 11

FIG. 12

FIG. 13

FIG. 14

FIG. 15

**FIG. 16**

**FIG. 17**

24

**FIG. 18**

Europäisches
Patentamt

European
Patent Office

Office européen
des brevets

# EUROPEAN SEARCH REPORT

Application Number

EP 23 38 2433

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/228024 A1 (BATZER ULRICH [DE] ET AL) 11 August 2016 (2016-08-11)<br>* paragraph [0063] – paragraph [0065] *<br>* figure 7 * | 1-15 | INV.<br>A61B5/305 |
| A | EP 3 821 799 A1 (SHENZHEN MINDRAY BIOMEDICAL ELECTRONICS CO LTD [CN] ET AL.) 19 May 2021 (2021-05-19)<br>* paragraph [0038] – paragraph [0044] *<br>* figure 6 * | 1-15 | |
| A | US 2017/099528 A1 (BATZER ULRICH [DE]) 6 April 2017 (2017-04-06)<br>* paragraphs [0061] – [0074] *<br>* figures 8, 9 * | 1-15 | |

TECHNICAL FIELDS
SEARCHED    (IPC)

A61B

The present search report has been drawn up for all claims

| Place of search | Date of completion of the search | Examiner |
|---|---|---|
| The Hague | 19 September 2023 | Van Dop, Erik |

EPO FORM 1503 03.82 (P04C01)

## ANNEX TO THE EUROPEAN SEARCH REPORT
## ON EUROPEAN PATENT APPLICATION NO.

EP 23 38 2433

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2023

| Patent document cited in search report | | Publication date | Patent family member(s) | | Publication date |
|---|---|---|---|---|---|
| US 2016228024 | A1 | 11-08-2016 | CN | 105852844 A | 17-08-2016 |
| | | | DE | 102015202447 A1 | 11-08-2016 |
| | | | US | 2016228024 A1 | 11-08-2016 |
| EP 3821799 | A1 | 19-05-2021 | CN | 112312837 A | 02-02-2021 |
| | | | EP | 3821799 A1 | 19-05-2021 |
| | | | WO | 2020010580 A1 | 16-01-2020 |
| US 2017099528 | A1 | 06-04-2017 | CN | 107019509 A | 08-08-2017 |
| | | | DE | 102015219037 A1 | 06-04-2017 |
| | | | US | 2017099528 A1 | 06-04-2017 |

EPO FORM P0459

For more details about this annex : see Official Journal of the European Patent Office, No. 12/82